# DEMANDE DE BREVET EUROPEEN

(11) **EP 1 095 589 A1**
(43) Date de publication de la demande: **02.05.2001**
(21) Numéro de dépôt: 00420223.0
(22) Date de dépôt: 27.10.2000
(51) Int. Cl.: A45D 44/00, A61K 7/48

(54) **Ensemble de pochette et masque pour soin dermatologique ou cosmétique**

(30) Priorité: 27.10.1999 FR 9913688
(71) Demandeur: Progress, 69100 Villeurbanne (FR)
(72) Inventeur: Samec, Jean-Marie, 01250 Ceyzeriat (FR); Perdrix, Catherine, 01250 Ceyzeriat (FR)
(74) Mandataire: Guerre, Dominique

(57) **Abrégé**

L'ensemble de pochette (1) et masque (2) de soin dermatologique ou cosmétique est caractérisé par un masque (2) en matériau imprégnable, dans sa forme déployée, et prêt à l'emploi, et une composition (7) dermatologique et/ou cosmétique imprégnant le masque, ladite composition constituant le seul milieu avec lequel le masque est en contact, elle est en matière étanche et indéchirable, permettant de maintenir le masque (2) dans sa forme déployée, elle est hermétiquement fermée.

## Description

La présente invention concerne une pochette de soin dermatologique ou cosmétique pour le traitement du visage.

Le document EP-A-0 063 875 décrit un masque facial en textile qui est imprégné d'un agent cosmétique et qui présente des évidements correspondant aux yeux, au nez et à la bouche. L'utilisateur l'applique directement sur son visage sur lequel le textile imbibé adhère jusqu'à la fin du temps de contact nécessaire au traitement. Le masque est ensuite retiré et le visage est éventuellement nettoyé, rincé et/ou séché.

Si cet art antérieur peut faciliter l'accès pour l'utilisateur à des traitements classiquement effectués en institut de beauté, il n'apporte aucune solution à la disponibilité du masque imprégné, en particulier dans le commerce, ni à sa conservation au cours de son stockage.

En effet, l'agent cosmétique qui imbibe le masque doit être maintenu à l'abri de toute dégradation, et notamment oxydante, au cours du stockage du masque. Ainsi, selon le traitement visé, une application hebdomadaire, voire quotidienne sur une période déterminée, du masque peut se révéler utile. Dans ce cas, l'utilisateur voudra disposer de plusieurs masques, mais ceux-ci doivent être conservés de manière à ce que l'efficacité de l'agent cosmétique soit totalement préservée.

En outre, il peut être particulièrement avantageux pour l'utilisateur de choisir un masque en fonction de la forme de son visage.

On connaît selon US-A-3 499 446, un masque pour le traitement du visage ou d'une autre partie du corps, qui est en un matériau poreux imprégné d'une composition cosmétique. Celle-ci est appliquée à l'état liquide sur le matériau, mais avant son utilisation, elle est à l'état solide. A cet effet, elle incorpore, en plus des ingrédients actifs, au moins, d'une part, un support pour que cette composition se présente à l'état solide à température ambiante, et ramollisse au contact de la partie à traiter, et, d'autre part, un solvant volatil qui, une fois que la composition est exposée à l'air, entraînera son séchage rapide. Ce séchage se matérialise par une rétraction du masque.

Ce masque peut être conditionné dans un emballage.

Ce masque présente plusieurs inconvénients. D'abord, la composition comprend d'autres ingrédients que ceux strictement nécessaires aux soins et peut entraîner des effets secondaires notamment irritation de la peau traitée, ensuite le séchage de la composition entraîne un inconfort, surtout quand la partie à traiter est le visage.

La présente invention apporte une solution qui permet de résoudre les problèmes posés par les masques connus, avec un ensemble de pochette et masque de soin dermatologique ou cosmétique, répondant aux caractéristiques suivantes:
la pochette contient un masque en matériau imprégnable, dans sa forme déployée,
le masque est imprégné d'une composition dermatologique ou cosmétique et est prêt à l'emploi, ladite composition constituant le seul milieu avec lequel le masque est en contact dans la pochette,
elle est en matière étanche et indéchirable, et permet de maintenir le masque dans sa forme déployée, et
elle est hermétiquement fermée.

Cette présentation individuelle d'un masque permet tout à la fois de le présenter de manière visible, de protéger la composition dermatologique ou cosmétique de toute dégradation vis-à-vis d'agents chimiques et/ou physiques et de toute variation du pH, et de maintenir le masque dans un milieu où la proportion initiale de la composition ne change pas.

Ainsi, par « prêt à l'emploi », on entend un masque qui ne nécessite aucun traitement avant ou après son retrait de la pochette ; en particulier, la composition dermatologique ou cosmétique n'est constituée que des ingrédients strictement nécessaires au soin.

La pochette peut être manipulée, rangée, voire pliée si nécessaire, sans risque d'altération du masque.

La matière étanche et indéchirable de la pochette doit maintenir le masque dans sa forme déployée, quelles que soient les contraintes imposées à la pochette. Elle est de préférence transparente pour permettre de visualiser le masque, elle peut aussi être de couleur. Cette matière peut aussi être opaque à au moins une partie des rayonnements lumineux, de façon à protéger une composition dermatologique ou cosmétique qui serait particulièrement sensible à la lumière.

De préférence, la matière de la pochette est une matière plastique, par exemple un polyéthylène tel qu'un polyéthylène basse densité ou un chlorure de polyvinyle.

Le matériau imprégnable du masque peut être un textile, tissé ou non. Avantageusement il est en coton, de préférence en coton tissé.

Un masque préféré est adapté au visage et présente au moins un évidement et des découpes prévues pour les narines. Ces découpes permettent une application facile et confortable du masque sur les ailes du nez et des pommettes. Mais selon la nature de la composition dermatologique ou cosmétique, et en particulier si la composition est susceptible d'entraîner une irritation des paupières ou des lèvres, il conviendra de prévoir en outre des évidements correspondant à la bouche et/ou aux yeux.

Bien entendu, un masque de l'invention peut être adapté à toute autre partie du corps.

Un ensemble particulièrement avantageux de l'invention est destiné à un traitement anti-ride et hydratant et la pochette contient à cet effet un masque imprégné d'une composition à base de collagène et d'élastine.

Selon l'ensemble de l'invention, la pochette est hermétiquement scellée par toute technique classique, telle que par soudage par résistance à haute fréquence. Il est préférable que la fermeture de la pochette s'effectue dans des conditions stériles, de manière à éviter tout risque de contamination, qui affecterait l'intégrité du masque.

La pochette est alors ouverte à l'aide d'une paire de ciseaux.

On peut également prévoir une fermeture hermétique de la pochette que l'on peut rompre plus facilement, c'est-à-dire qui ne nécessiterait pas d'outils.

Avec l'objet de l'invention, l'utilisateur a à sa disposition un masque prêt à l'emploi, dont le retrait de sa pochette peut être effectué juste au moment de l'application. La composition dermatologique ou cosmétique qui subsisterait après retrait du masque peut être recueillie par l'utilisateur afin qu'il l'applique par exemple sur son cou, ses membres.

Les pochettes de l'invention peuvent être regroupées dans des emballages pour leur commercialisation.

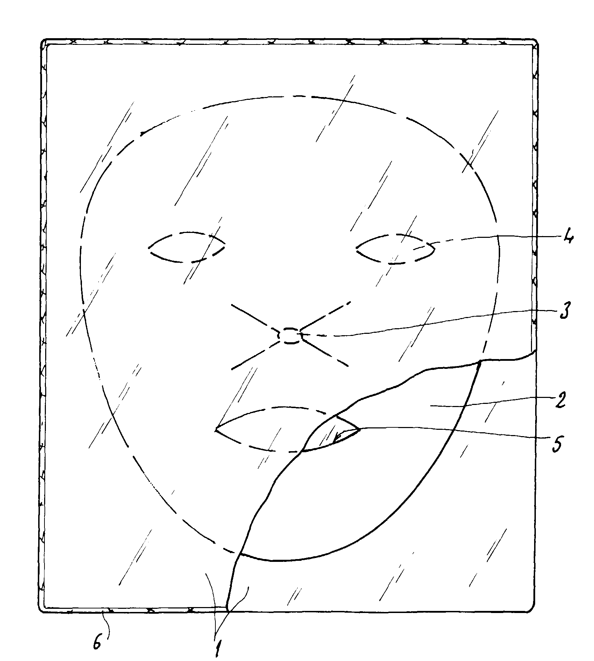

La figure 1 présente une pochette de soin de l'invention.

La pochette 1 contient un masque 2 imbibé d'une composition cosmétique ou dermatologique 7. Le masque présente un évidement et des découpes 3 pour les narines et des évidements 4 et 5, pour respectivement les yeux et la bouche. La pochette est hermétiquement fermée par des soudures 6.

## Revendications

1. Ensemble de pochette (1) et masque (2) de soin dermatologique ou cosmétique, **caractérisé en ce que** le masque (2) est en matériau imprégnable, dans sa forme déployée, et en ce qu'il est imprégné d'une composition (7) dermatologique et/ou cosmétique, et est prêt à l'emploi, ladite composition constituant le seul milieu avec lequel le masque est en contact, et en ce que la pochette, en matière étanche et indéchirable, permet de maintenir le masque dans sa forme déployée, et est hermétiquement fermée.

2. Ensemble selon la revendication 1, caractérisé en ce que la matière de la pochette est transparente.

3. Ensemble selon la revendication 1 ou 2, caractérisé en ce que la matière de la pochette est opaque à au moins une partie des rayonnements lumineux.

4. Ensemble selon l'une quelconque des revendications 1 à 3, caractérisé en ce que la pochette est en matière plastique.

5. Ensemble selon l'une quelconque des revendications 1 à 4, caractérisé en ce que le masque (2) est en coton tissé.

6. Ensemble selon l'une quelconque des revendications 1 à 5, caractérisé en ce que le masque (2) présente au moins un évidement et des découpes (3) prévues pour les narines.

7. Ensemble selon la revendication 6, caractérisé en ce que le masque (2) présente en outre au moins un évidement prévu pour la bouche (5) et/ou les yeux (4).

8. Ensemble selon l'une quelconque des revendications 1 à 7, caractérisé en ce que la composition (7) est à base de collagène et d'élastine.
